# EUROPÄISCHE PATENTSCHRIFT

(11) **EP 3 981 336 B1**
(45) Veröffentlichungstag und Bekanntmachung des Hinweises auf die Patenterteilung: **07.06.2023**
(21) Anmeldenummer: 21211589.3
(22) Anmeldetag: 15.09.2017
(51) Int. Cl.: A61B 10/02

(54) **BIOPSIEPISTOLE**
BIOPSY GUN
PISTOLET DE BIOPSIE

(30) Priorität: 16.09.2016 DE 202016105165 U
(43) Veröffentlichungstag der Anmeldung: 13.04.2022
(62) Teilanmeldung aus: 17772642.9
(73) Patentinhaber: Peter Pflugbeil GmbH, 85604 Zorneding (DE)
(72) Erfinder: KNEISSL, Florian, 80639 München (DE)
(74) Vertreter: TBK

(56) Entgegenhaltungen:
- DE-A1-102008 038 414
- DE-U1-202011 004 277
- US-A1- 2012 004 572
- US-A1- 2015 148 704

## Beschreibung

Aus dem Stand der Technik ist eine handspannbare Biopsiepistole bekannt, bei der eine Kanüle und eine in der Kanüle geführtes Stilett mittels zwei aufeinanderfolgenden Hüben eines Spanngriffs gespannt werden können. Zu diesem Zweck ist die Kanüle an einem Kanülenschlitten befestigt und das Stilett ist an einem Stilettschlitten befestigt, und beide Schlitten sind in einem Gehäuse der Biopsiepistole nebeneinander angeordnet und in der Längsrichtung eines Gehäuses der Biopsiepistole in dem Gehäuse verschiebbar gelagert. Der Spanngriff hat ein längliches Element, das drehfest an dem Spanngriff angebracht ist und seitlich gebogen werden kann. Am vorderen Ende des länglichen Elements sind seitlich zwei Zähne ausgebildet, die in entsprechende Vertiefungen des Kanülenschlittens bzw. des Stilettschlittens eingreifen können.

Im entspannten Zustand von Kanülenschlitten und Stilettschlitten ist der entsprechenden Zahn des länglichen Elements mit der Vertiefung des Kanülenschlittens in Eingriff, sodass während des Spannens des Kanülenschlittens das längliche Element in Querrichtung gebogen wird, damit der der Vertiefung des Stilettschlittens entsprechende Zahn an der Vertiefung des Stilettschlittens vorbeigeführt werden kann und im ersten Hub nur der Kanülenschlitten, nicht aber der Stilettschlitten vorgespannt wird.

Nachdem der Kanülenschlitten vorgespannt wurde und der Spanngriff auf seine entspannte Ausgangsposition zurückkehrt, stellt sich auch der verbogenen Zustand des länglichen Elements zurück, sodass im zweiten Hub der andere Zahn, der der Vertiefung des Stilettschlittens entspricht, mit dieser in Eingriff gelangt und somit der Stilettschlitten vorgespannt werden kann.

Damit das längliche Element in Querrichtung leicht genug gebogen werden kann, ist es erforderlich, dass es eine Mindestlänge hat. Dies führt dazu, dass die gesamte Biopsiepistole eine Mindestlänge hat, die es erschweren kann, die Biopsiepistole auf bequeme Weise einhändig vorzuspannen.

Es ist daher eine erste Aufgabe der vorliegenden Erfindung, eine verbesserte Biopsiepistole mit geringerer Länge bereitzustellen.

Außerdem haben solche Biopsiepistolen üblicherweise zwei Taster, nämlich einen ersten Taster für das alleinige Auslösen des Stiletts und einen zweiten Taster für nachgeschaltete Auslösen der Kanüle, falls das Stilett bereits ausgelöst wurde, oder für das zeitversetzte Auslösen von Stilett und Kanüle. Um dem zweiten Taster zu ermöglichen, nicht nur die Kanüle, sondern auch das Stilett auszulösen, ist entweder ein weiteres Bauteil erforderlich, oder ein Schnapphaken des Stilettschlitten muss so gestaltet werden, dass er nicht nur für den ersten Taster sondern auch für den zweiten Taster erreichbar ist. Beide Varianten machen den Aufbau der Biopsiepistole kompliziert.

Aus der US 2015/148704 A1 ist eine Biopsiepistole gemäß dem Oberbegriff von Anspruch 1 bekannt. US 2012/004572 A1, DE 102008038414 A1 und DE 202011004277 U1 zeigen weitere bekannte Biopsiepistolen.

Es ist daher eine zweite Aufgabe der vorliegenden Erfindung, eine verbesserte Biopsiepistole mit weniger kompliziertem Aufbau bereitzustellen.

Es ist eine dritte Aufgabe der vorliegenden Erfindung, eine Biopsiepistole zu schaffen, die leicht zusammengebaut werden kann und die dennoch stabil ist. Eine vierte Aufgabe der vorliegenden Erfindung besteht darin, eine Biopsiepistole zu schaffen, die auch dann, wenn Bauteile mit relativ geringer Steifigkeit zum Einsatz kommen, sicher vorgespannt und ausgelöst werden kann.

Eine fünfte Aufgabe der Erfindung besteht darin, eine Biopsiepistole zu schaffen, die sicher vorgespannt werden kann.

Erfindungsgemäß werden diese Aufgaben jeweils mit den Schutzansprüchen 1, 5, 8, 9 und 12 gelöst.

Im folgende wird ein Ausführungsbeispiel der Erfindung unter Bezugnahme auf die folgenden Figuren beschrieben, in welchen:
Fig. 1 eine Perspektivansicht einer Biopsiepistole gemäß dem Ausführungsbeispiel ist;
Fig. 2a eine Perspektivansicht der Biopsiepistole ohne obere Gehäusehalbschale und ohne Spanngriff ist;
Fig. 2b eine Perspektivansicht eines Rahmenelements der unteren Gehäuseschale ist;
Fig. 3a eine Draufsicht der oberen Gehäusehalbschale ist, und Fig. 3b eine Unteransicht der oberen Gehäusehalbschale ist;
Fig. 4 eine Draufsicht der Biopsiepistole ohne obere Gehäusehalbschale aber mit Spanngriff ist; Fig. 4a eine Draufsicht eines Eingriffselement ist; Fig. 4b eine Perspektivansicht des Spanngriffs ist; Fig. 4c eine Perspektivansicht eines Zapfens ist;
Fig. 5 eine perspektivische Innenansicht der oberen Gehäusehalbschale ist;
Fig. 6 eine Draufsicht eines Abschnitts der Biopsiepistole ohne obere Gehäusehalbschale in dem Zustand zeigt, in dem sich der Spanngriff in der Ausgangsposition befindet;
Fig. 7 eine Draufsicht einen Abschnitts der Biopsiepistole ohne obere Gehäusehalbschale in dem Zustand zeigt, in dem der Spanngriff in einem ersten Hub geringfügig vorgerückt wurde, sodass ein Kanülenschlitten gespannt wird;
Fig. 8 eine Draufsicht eines Abschnitts der Biopsiepistole ohne obere Gehäusehalbschale in dem Zustand zeigt, in dem der Kanülenschlitten gespannt wurde und der Spanngriff auf seine Ausgangsposition zurückgekehrt ist;

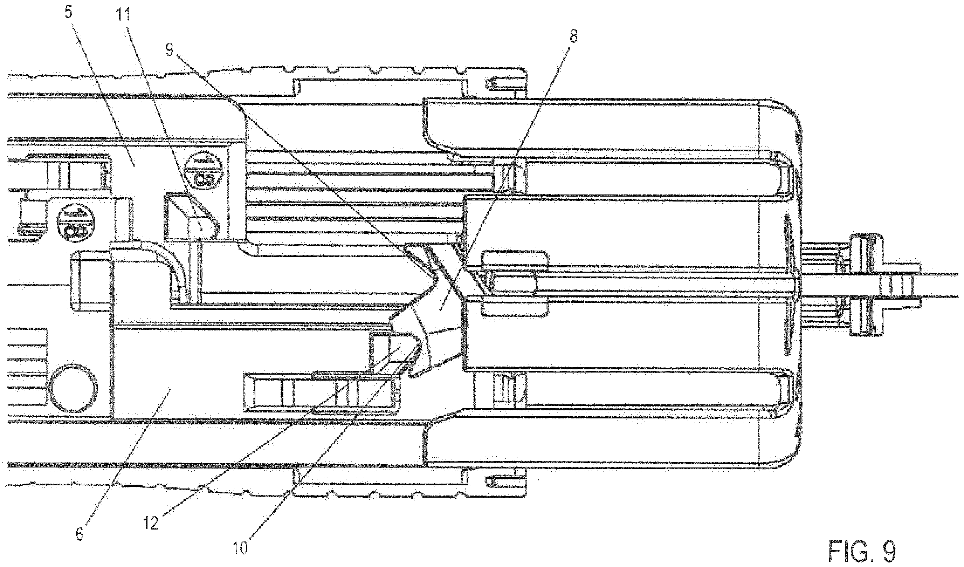

Fig. 9 eine Draufsicht eines Abschnitts der Biopsiepistole ohne obere Gehäusehalbschale in dem Zustand zeigt, in dem der Spanngriff in einem zweiten Hub geringfügig vorgerückt wurde, sodass ein Stilettschlitten gespannt wird;

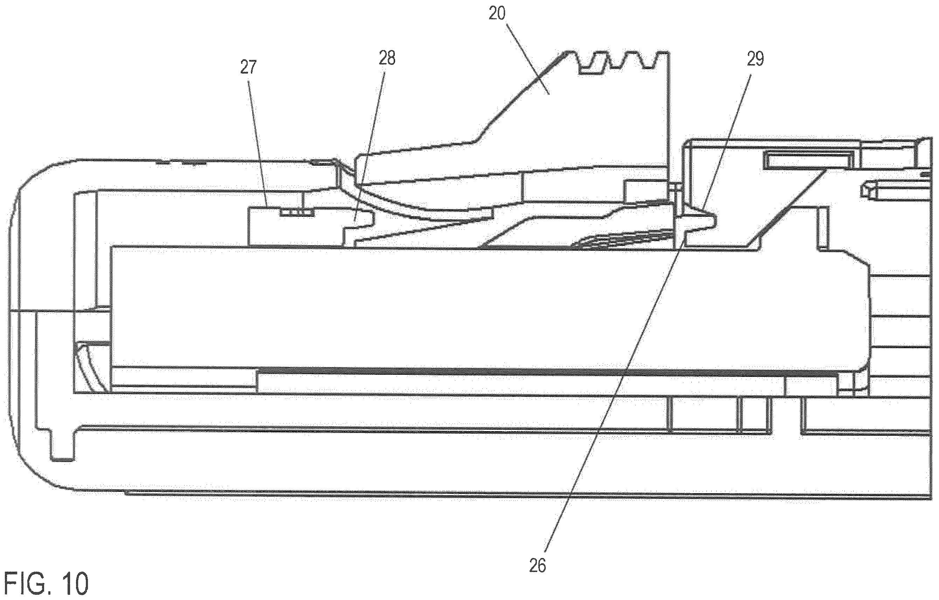

Fig. 10 einen horizontalen Längsschnitt durch die Biopsiepistole in einem Zustand zeigt, in dem der Stilettschlitten gespannt ist;
Fig. 11 einen horizontalen Längsschnitt durch die Biopsiepistole in einem Zustand zeigt, in dem der Stilettschlitten ausgelöst wurde bzw. nicht gespannt ist;

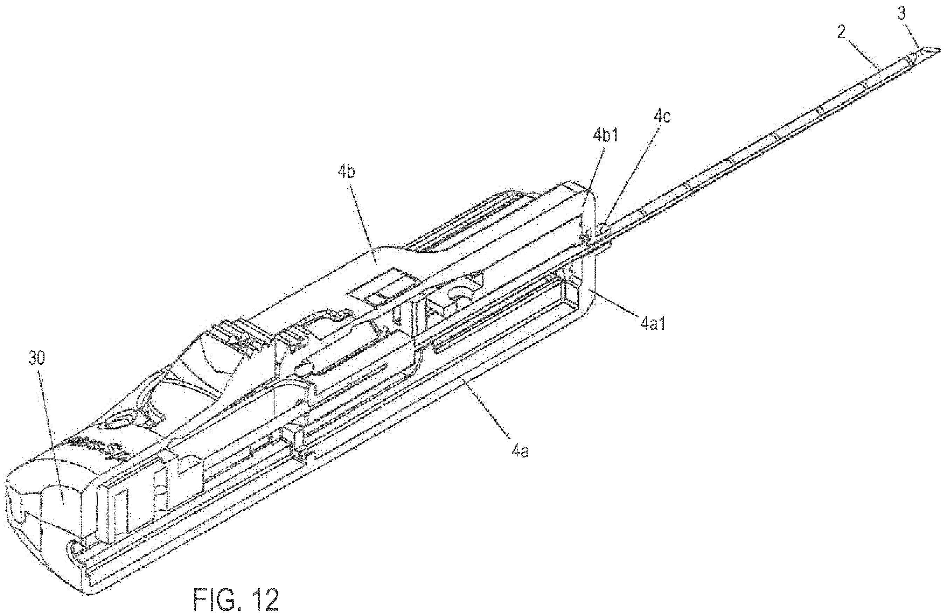

Fig. 12 einen vertikalen Längsschnitt durch die Biopsiepistole zeigt.

In der folgenden Beschreibung bezieht sich die Längsrichtung der Biopsiepistole auf die Richtung von Kanüle und Stilett, wobei die Seite mit Kanüle und Stilett die Vorderseite ist. Die Querrichtung verläuft in der Richtung, in der Kanülenschlitten und Stilettschlitten nebeneinander angeordnet sind, und die Vertikalrichtung verläuft senkrecht zu diesen beiden Richtungen, wobei die Seite mit den Tastern als die Oberseite bezeichnet wird.

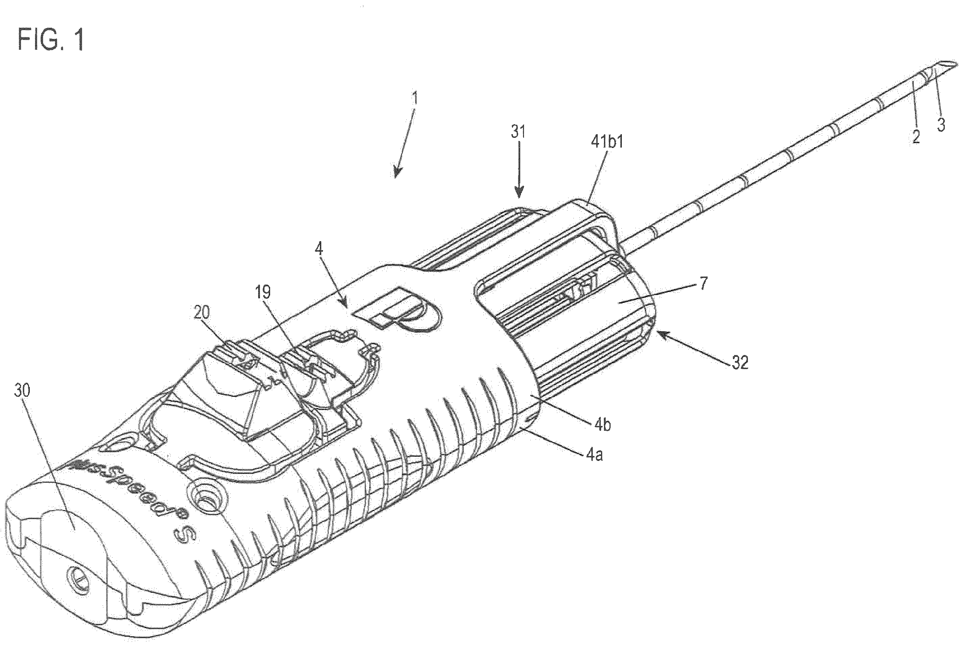

Fig. 1 zeigt eine Biopsiepistole 1 mit einem Gehäuse 4, einem Spanngriff 7, einer Kanüle 2 und einem Stilett 3. Kanüle 2 und Stilett 3 lassen sich durch zweimaliges Betätigen des Spanngriffs 7 getrennt vorspannen. Der Spanngriff 7 ist durch zwei Öffnungen 31, 32 des Gehäuses 4 für Zeigefinger und Mittelfinger einer Hand eines Anwenders zugänglich, während der Daumen des Anwenders in der dafür vorgesehenen Vertiefung 30 am anderen Ende des Gehäuses 4 abrutschsicher platziert wird. Das Gehäuse 4 besteht im Wesentlichen aus zwei Halbschalen 4a, 4b, und zwischen den Öffnungen 31, 32 des Gehäuses 4 befindet sich jeweils L-förmige Rahmenelemente 4a1, 4b1 der beiden Halbschalen 4a, 4b. Ferner sind in Fig. 1 ein erster Taster 19 zum Auslösen des Stiletts und ein zweiter Taster 20 zum Auslösen der Kanüle gezeigt.

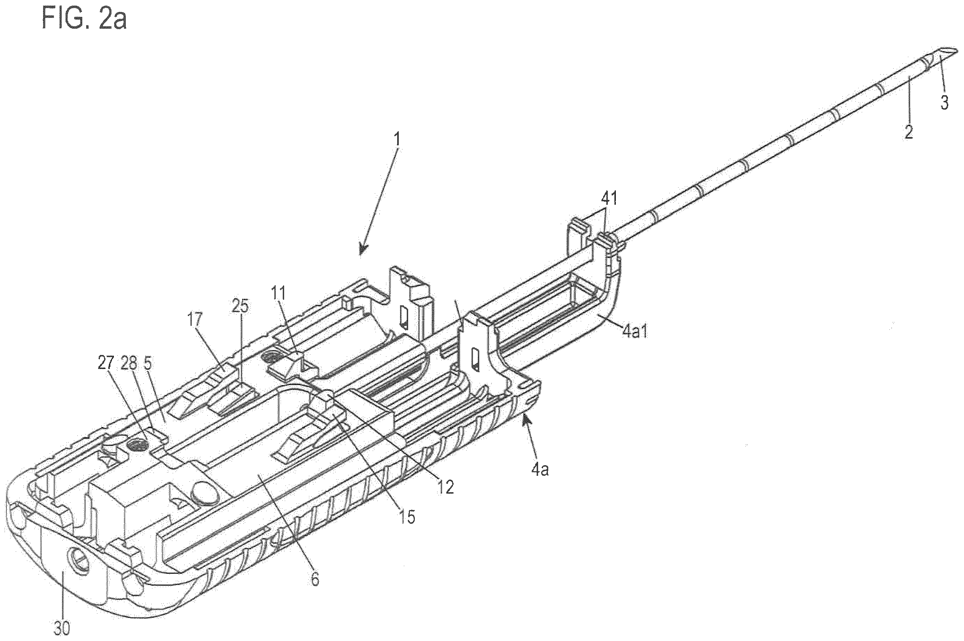

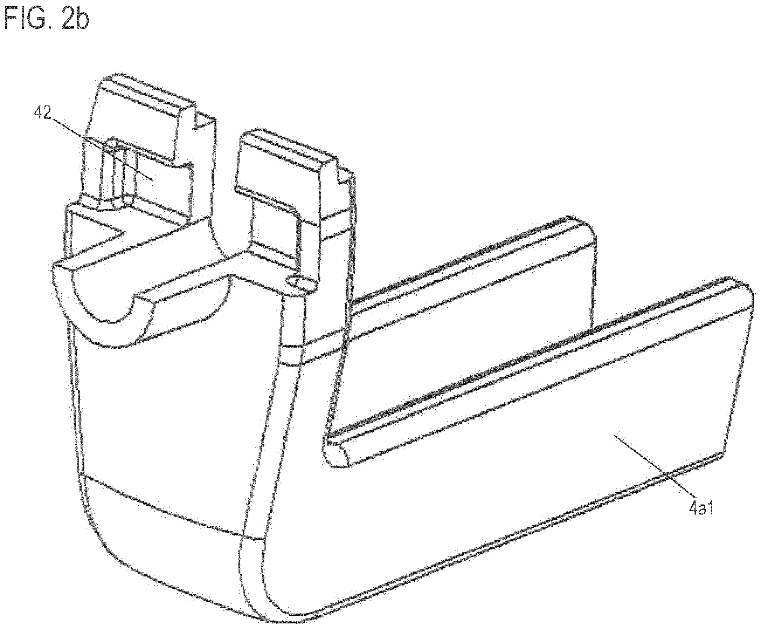

Fig. 2 zeigt die Biopsiepistole 1 ohne Spanngriff 7 und ohne obere Gehäusehalbschale 4b. Ferner hat die Biopsiepistole 1 einen Kanülenschlitten 5, der mit der Kanüle 2 verbunden ist, und einen Stilettschlitten 6, der mit dem Stilett 3 verbunden ist. Beide Schlitten können Spritzbauteile sein, die direkt an Kanüle bzw. Stilett angespritzt sind. Die Schlitten sind auf jeweiligen Schienen (nicht gezeigt) geführt und sind durch jeweilige Schraubenfedern (nicht gezeigt) nach vorne vorgespannt.

Der Kanülenschlitten 5 hat einen kanülenseitigen Vorsprung 11, der von der oberen Fläche des Kanülenschlittens 5 nach oben vorragt. In diesem Ausführungsbeispiel ist der kanülenseitige Vorsprung 11 an seiner Vorderseite abgerundet und er hat nach hinten und außen stabilisierende Schrägflächen.

Weiter hinten hat der Kanülenschlitten 5 an seiner in Querrichtung äußeren Seiten einen ersten Kanülenschnapphaken 17 und an seiner in Querrichtung inneren Seiten einen zweiten Kanülenschnapphaken 25. Beide Kanülenschnapphaken 17, 25 sind in Vertikalrichtung nachgiebig befestigt, sodass sie in Vertikalrichtung heruntergedrückt werden können. Diese nachgiebige Verbindung entsteht dadurch, dass die Kanülenschnapphaken 17, 25 auskragend und einstückig an dem Kanülenschlitten 5 ausgebildet sind.

Der Stilettschlitten 6 hat einen stilettseitigen Vorsprung 12, der von der oberen Fläche des Kanülenschlittens 6 nach oben vorragt. In diesem Ausführungsbeispiel ist der stilettseitige Vorsprung 12 an seiner Vorderseite abgerundet und er hat nach hinten und außen stabilisierende Schrägflächen.

Weiter hinten hat der Stilettschlitten 6 einen Stilettschnapphaken 15, der in Vertikalrichtung nachgiebig befestigt ist, sodass er in Vertikalrichtung heruntergedrückt werden kann. Diese nachgiebige Verbindung entsteht dadurch, dass der Stilettschnapphaken 15 auskragend und einstückig an dem Stilettschlitten 6 ausgebildet ist.

Außerdem hat der Stilettschlitten 6 ein Auslöseelement 27 mit einer nach vorne vorragenden Nase 28, die sich mit Bezug auf die Querrichtung auf einer gemeinsamen Längsachse mit dem zweiten Kanülenschnapphaken 25 befindet.

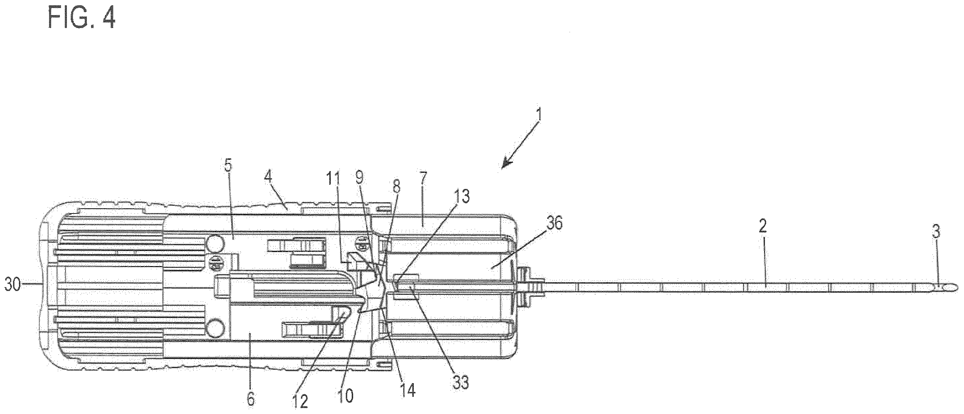

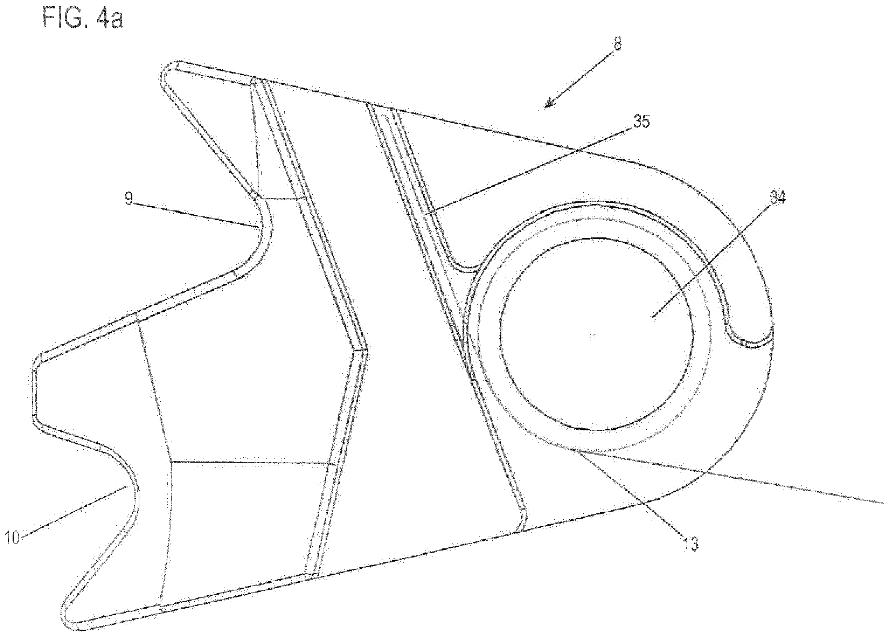

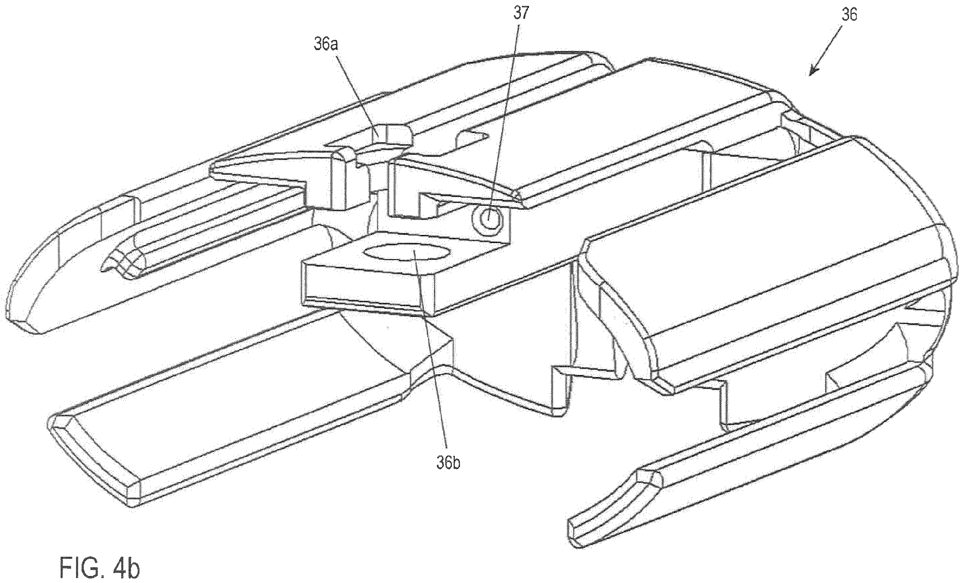

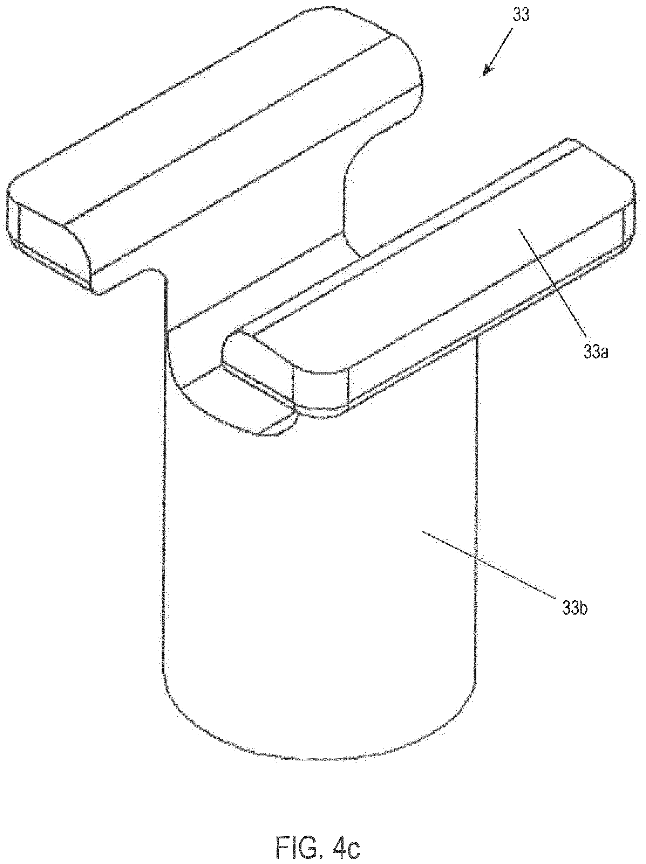

Fig. 4 zeigt die Biopsiepistole mit Spanngriff 7, an dem ein Eingriffselement 8 drehbar angebracht ist. Wie in Fig. 4a zu sehen ist, hat das Eingriffselement 8 in der Draufsicht eine im Wesentlichen dreiecksartige Form, deren vordere Spitze ein Durchgangsloch 34 hat und deren hinteren Spitzen eine kanülenseitige Vertiefung 9 und eine stilettseitige Vertiefung 10 hat. Ferner ist das Eingriffselement 8 im Bereich der Vertiefungen 9 und 10 stärker als im Bereich des Durchgangslochs 34. Zudem befindet sich im weniger starken Bereich um das Eingriffsloch 34 herum eine Federaufnahmenut 35, in der ein erstes Ende einer Schraubenfeder 13 untergebracht werden kann, während die Schraubenfeder 13 das Durchgangsloch 34 umgibt.

Fig. 4b zeigt einen Abschnitt 36 des Spanngriffs 7, der das Eingriffselement 8 drehbar trägt. Zu diesem Zweck hat der Abschnitt 36 eine rechteckige Aufnahme 36a in einem oberen Teil des Abschnitts 36 und ein Durchgansloch 36b in einem unteren Teil des Abschnitts 36. Zudem ist in dem Abschnitt 36 ein sich in Längsrichtung erstreckendes Federaufnahmeloch 37 ausgebildet, das sich in Vertikalrichtung zwischen dem oberen Teil und dem unteren Teil des Abschnitts 36 befindet und in welches ein zweites Federende eingeführt werden kann.

In Fig. 4c ist ein Zapfen 33 dargestellt, der einen zylindrischen unteren Abschnitt 33b und einen oberen Abschnitt 33a mit im Wesentlichen rechteckiger Kontur hat.

Das Eingriffselement 8 ist derart an dem Spanngriff 7 montiert, dass die Feder 13 das Durchgangsloch 34 umgibt und das erste Federende in der Federaufnahmenut 35 untergebracht ist. Ferner ist das zweite Federende in das Federaufnahmeloch 37 eingeführt und der zylindrische Abschnitt 33b des Zapfens 33 durchdringt das Durchgangsloch 34 des Eingriffselements 8 sowie das Durchgangsloch 36b des Abschnitts 36 während der obere Abschnitt 33b mit rechteckigen Kontur in der rechteckigen Aufnahme 36a aufgenommen ist. Dadurch ist das Eingriffselement 8 an dem Abschnitt 36 des Spanngriffs 7 drehbar gelagert, wobei das Eingriffselement 8 durch die Schraubenfeder 13 zunächst in Richtung des Stilettschlittens 6 vorgespannt ist.

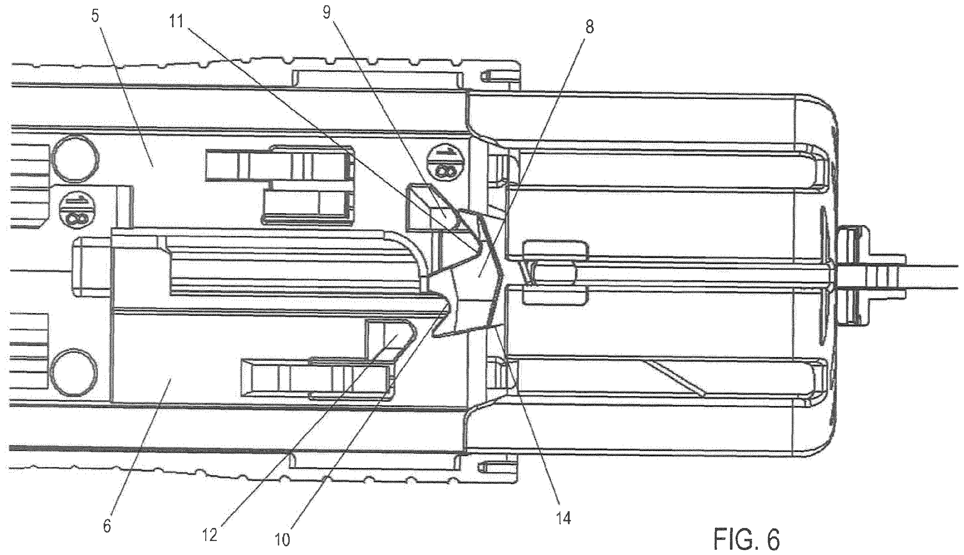

Ferner ist der Spanngriff 7 mit dem daran montierten Eingriffselement 8 derart an dem Gehäuse 4 montiert, dass das Eingriffselement 8 mit einer Anlagefläche 14 in Anlage kommt, wenn der Spanngriff durch eine Schraubenfeder (nicht gezeigt) in die Ausgangslage gespannt ist. Durch die Anlage des Eingriffselements 8 an der Anlagefläche 14 wird das Eingriffselement 8 in der Ausgangslage des Spanngriffs 7 gegen die Kraft der Feder 13 von dem Stilettschlitten weg und in Richtung zu dem Kanülenschlitten hin gedreht. Diese Position ist vergrößert in Fig. 6 dargestellt.

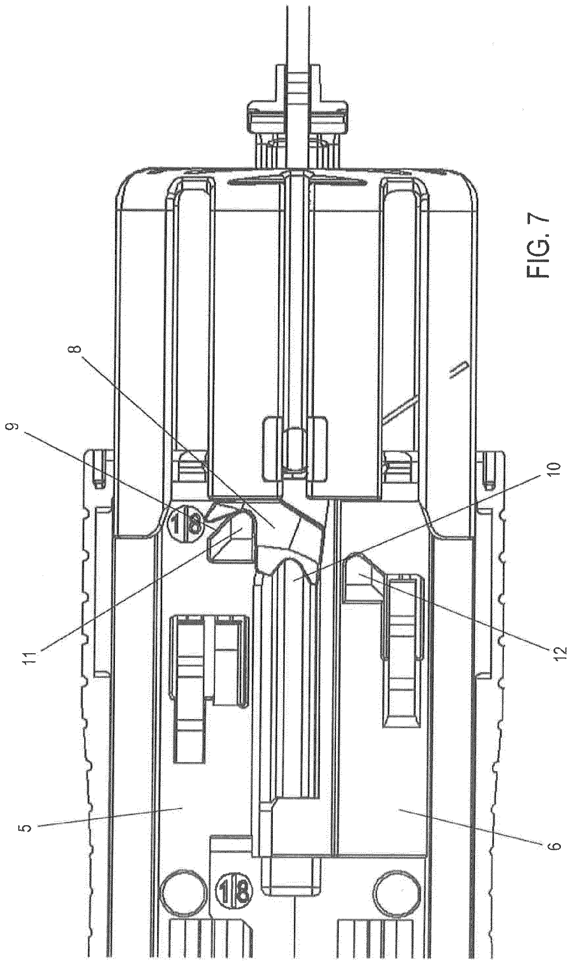

Wie in Fig. 6 zu sehen ist, ist der äußere Randbereich der Vertiefung 9 des Eingriffselements 8 in der Ausgangslage des Spanngriffs 7 mit dem kanülenseitigen Vorsprung 11 derart in Kontakt, dass das Eingriffselement 8 durch eine Betätigung des Spanngriffs 7 aus der Ausgangslage heraus gegen die Kraft der Feder 13 weiter zu dem Kanülenschlitten 5 gedreht wird. Dadurch kommt die Vertiefung 9 mit dem kanülenseitigen Vorsprung 11 sicher in Eingriff (siehe Fig. 7), sodass der Kanülenschlitten 5 durch Bewegen des Spanngriffs 7 mitbewegt wird.

Wie zudem in Fig. 7 gezeigt ist, ermöglicht die Verdrehung des Eingriffselements 8 in Richtung Kanülenschlitten, dass das Eingriffselement 8 sicher an dem stilettseitigen Vorsprung 12 vorbei geführt werden kann, ohne damit in Eingriff zu gelangen. Damit wird sichergestellt, dass bei diesem ersten Hub lediglich der Kanülenschlitten 5 gespannt wird.

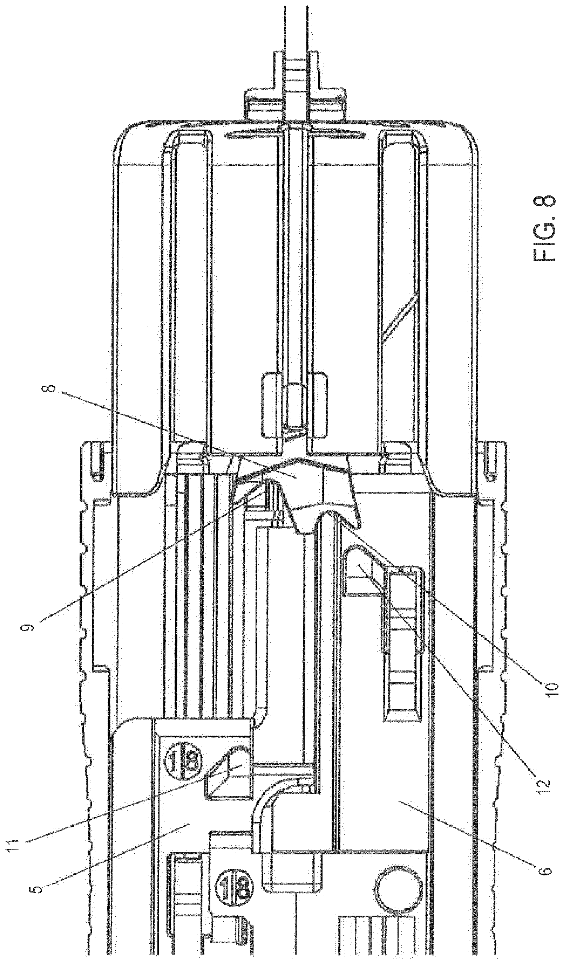

Nachdem der Kanülenschlitten 5 gespannt und mit dem Gehäuse 4 verrastet wurde (wird später genauer beschrieben), kehrt der Spanngriff 7 auf seine Ausgangslage zurück, die in Fig. 8 gezeigt ist. Da nun der Kanülenschlitten 5 gespannt ist, kommt die Vertiefung 9 nicht mehr mit dem kanülenseitigen Vorsprung 11 in Eingriff, sodass bei einer erneuten Spannbewegung des Spanngriffs 7 das Eingriffselement 8 mit dem Verlassen der Anlagefläche 14 durch die Kraft der Feder 13 in Richtung des Stilettschlittens 6 gedreht wird. Daher kann nun die Vertiefung 10 des Eingriffselement 8 mit dem stilettseitigen Vorsprung 12 in Eingriff gelangen, sodass der Stilettschlitten 6 mit der Bewegung des Spanngriffs 7 gespannt und mit dem Gehäuse 4 verrastet werden kann.

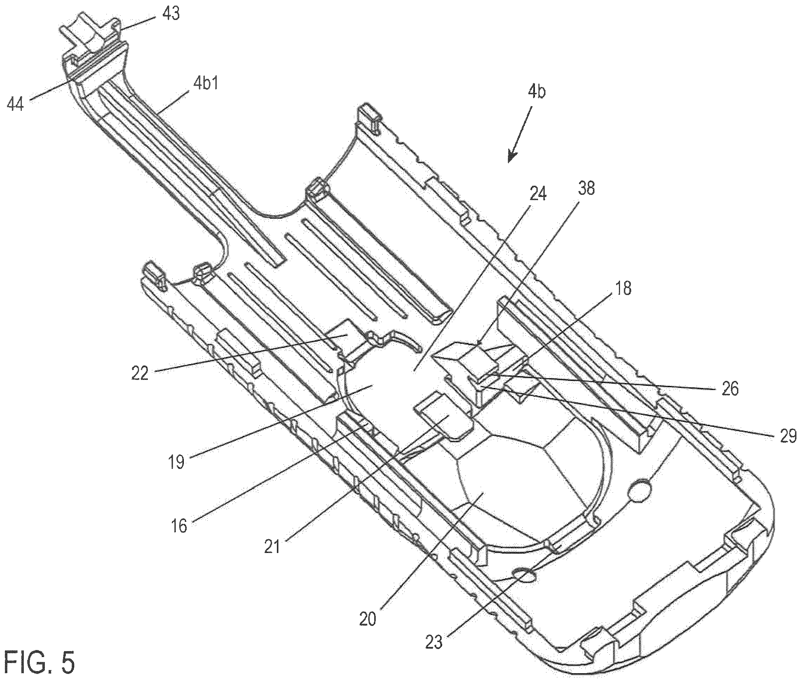

Nun wird das Einrasten der gespannten Schlitten an der Gehäuseoberschale 4b beschrieben. Wie in der Fig. 5 zu sehen ist, hat die Innenseite der oberen Gehäusehalbschale 4b in Querrichtung neben dem ersten Taster 19 zwei nach unten vorragende Bauteile, die in ihrem Sockelbereich einstückig ausgebildet sind. Das in Querrichtung außen liegende Bauteil bildet eine erste Kanülenschlittenhaltefläche 18 für den ersten Kanülenschnapphaken 17 (siehe Fig. 2a). Das in Querrichtung innen liegende Bauteil bildet eine zweite Kanülenschlittenhaltefläche 26 für den zweiten Kanülenschnapphaken 25 (siehe Fig. 2a). Beide Halteflächen 18 und 26 sind zur Rückseite gerichtet und verlaufen im Wesentlichen in Vertikalrichtung. Ferner haben beide Bauteile zur Vorderseite abgeschrägte Flächen, entlang denen die Kanülenschnapphaken 17, 25 beim jeweiligen Spannen gleiten und somit nach unten gedrückt werden, bis deren Vorderseiten hinter die Halteflächen 18, 26 gelangen und an diesen einrasten.

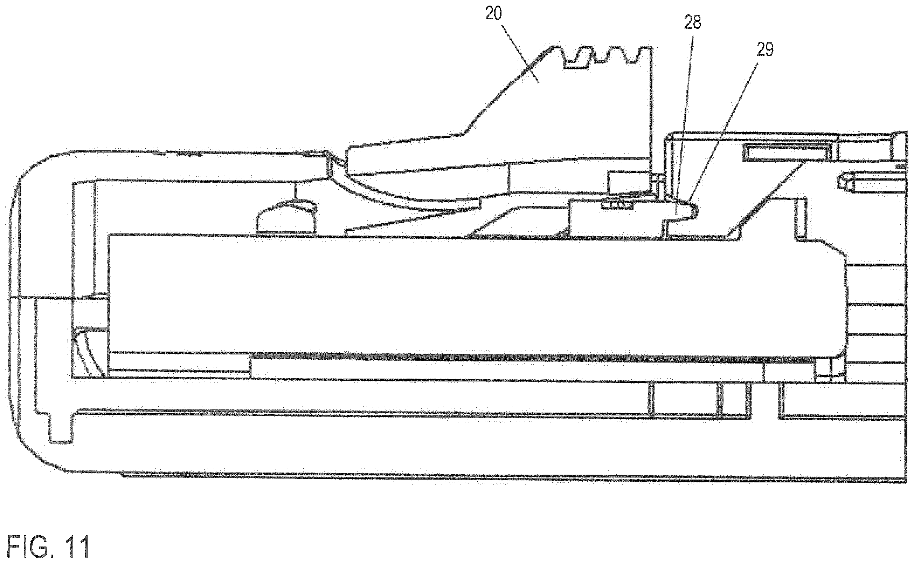

Außerdem hat das Bauteil, das die zweite Kanülenschlittenhaltefläche 26 bildet, eine Vertiefung 29, die über der zweiten Kanülenschlittenhaltefläche 26 ausgebildet ist und in Richtung der Vorderseite vertieft ist. In diese Vertiefung 29 ist die Nase 28 des Auslöseelement 27 eingetaucht, wenn sich der Kanülenschlitten 5 in seiner nicht gespannten Ausgangsposition befindet (siehe Fig. 11).

Wie ebenso in der Fig. 5 zu sehen ist, hat die Innenseite der oberen Gehäusehalbschale 4b in Querrichtung neben dem ersten Taster 19 an der den Kanülenschlittenhalteflächen 18, 26 entgegengesetzten Seite ein nach unten vorragendes Bauteil, das eine Stilettschlittenhaltefläche 16 für den Silettschnapphaken 15 (siehe Fig. 2) bildet. Die Haltefläche 16 ist zur Rückseite gerichtet und verläuft im Wesentlichen in Vertikalrichtung. Ferner hat das vorragende Bauteil, das die Stilettschlittenhaltefläche 16 bildet, eine zur Vorderseite abgeschrägte Fläche entlang der der Stilettschnapphaken 15 beim Spannen gleitet und somit nach unten gedrückt wird, bis dessen Vorderseiten hinter die Haltefläche 16 gelangt und an dieser einrastet.

Die gespannte Biopsiepistole 1 kann auf zwei Arten ausgelöst werden. Gemäß einer ersten Art wird zuerst der erste Taster 19 betätigt, sodass nur das Stilett 3 ausgelöst wird, und danach wird der zweite Taster 20 betätigt, um die Kanüle 2 auszulösen. Gemäß einer zweiten Art wird gleich der zweite Taster 20 betätigt, wodurch erst das Stilett 3 ausgelöst wird und danach, wenn sich das Stilett vollständig nach vorne bewegt hat, die Kanüle 2 ausgelöst wird.

Nun wird die erste Art des Auslösens beschrieben.

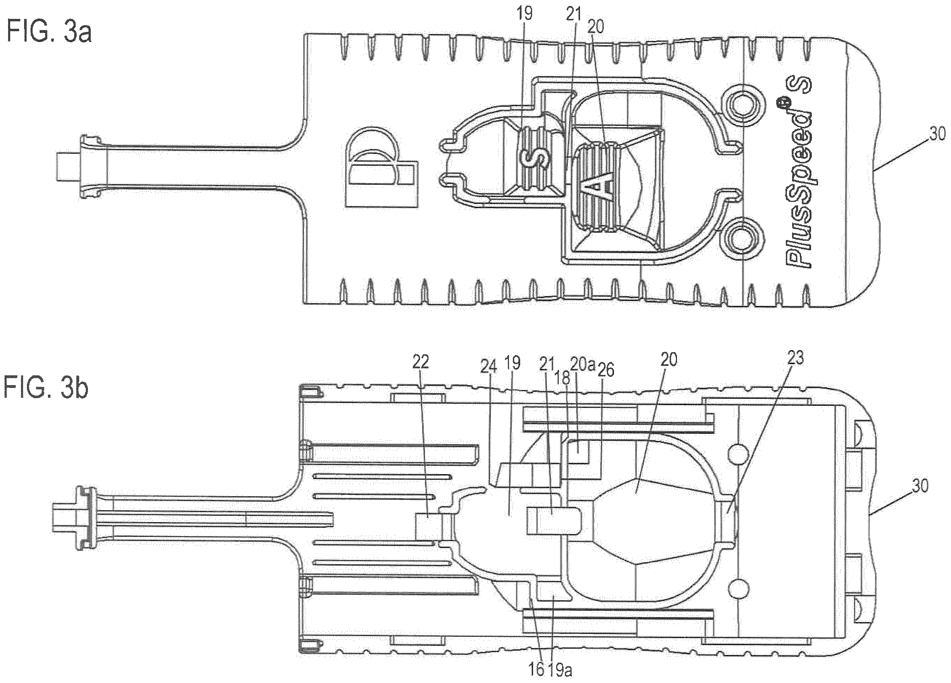

Wie in Figuren 3B und 5 zu sehen ist, ist der erste Taster 19 über ein Hauptverbindungselement 22 elastisch an dem Gehäuse 4 befestigt. Das Hauptverbindungselement 22 liegt an der Vorderseite des ersten Tasters 19, es verbindet den ersten Taster 19 einstückig mit dem Gehäuse 4 und es hat eine Querschnittsschwächung, die für die Elastizität sorgt.

Ferner ist der erste Taster 19 über ein zusätzliches Verbindungselement 24 einstückig mit dem Gehäuse 4 verbunden. Das zusätzliche Verbindungselement 24 liegt in etwa im Bereich der Bauteile, die die Halteflächen 18 und 26 bereitstellen, und damit in etwa in der auf die Längsrichtung bezogenen Mitte des ersten Tasters 19.

An der dem zusätzlichen Verbindungselement 24 in Querrichtung entgegengesetzten Seite des ersten Tasters 19 steht ein Auslösevorsprung 19a nach unten vor. Dieser Auslösevorsprung 19a wirkt im gespannten Zustand des Stilettschlittens 6 auf den Stilettschnapphaken 15, sodass bei Betätigung des ersten Tasters 19 die Vorderseite des Stilettschnapphakens 15 von der Stilettschlittenhaltefläche 16 gelöst wird und der Stilettschlitten 6 durch die Kraft der Schraubenfeder nach vorne bewegt wird.

In dem Zustand, in dem der Stilettschlitten 6 bereits nach vorne bewegt ist, der Kanülenschlitten 5 sich jedoch noch in seiner gespannten Position befindet, hat sich das Auslöseelement 27 des Stilettschlittens 6 über den ersten Kanülenschnapphaken 25 des Kanülenschlitten 5 bewegt und diesen nach unten gedrückt, sodass der Eingriff des Kanülenschnapphakens 25 an der Kanülenschlittenhaltefläche 26 gelöst wurde. Dadurch kann der Kanülenschlitten 5 erst dann ausgelöst werden, wenn das Stilett vollständig betätigt wurde.

Außerdem sorgt das zusätzliche Verbindungselement 24 dafür, dass der erste Taster 19 beim Betätigen um eine schräge Achse kippt, die im Wesentlichen durch das Hauptverbindungselement 22 und das zusätzliche Verbindungselement 24 verläuft, sodass der Auslösevorsprung 19a sicher nach unten gedrückt wird.

Wie in Figuren 3B und 5 zu sehen ist, ist der zweite Taster 20 über ein Hauptverbindungselement 23 elastisch an dem Gehäuse 4 befestigt. Das Hauptverbindungselement 23 liegt an der Hinterseite des zweiten Tasters 20, es verbindet den zweiten Taster 20 einstückig mit dem Gehäuse 4 und hat eine Querschnittsschwächung, die für die Elastizität sorgt.

An der dem Auslösevorsprung 19a des ersten Tasters 19 in Querrichtung entgegengesetzten Seite steht ein Auslösevorsprung 20a von dem zweiten Taster 20 nach unten vor. Dieser Auslösevorsprung 20a wirkt im gespannten Zustand des Kanülenschlittens 5 auf den Kanülenschnapphaken 17, sodass bei Betätigung des zweiten Tasters 20 die Vorderseite des Kanülenschnapphakens 17 von der Kanülenschlittenhaltefläche 18 gelöst wird und der Kanülenschlitten 5 durch die Kraft der Schraubenfeder nach vorne bewegt wird.

Nun wird die zweite Art des Auslösens beschrieben.

Wenn in dem Zustand, in dem beide Schlitten 5, 6 gespannt sind, der zweite Taster 20 betätigt wird, dann wirkt die Vorderseite des zweiten Tasters 20 auf eine Betätigungsnase 21 des ersten Tasters 19 ein, die sich unter den zweiten Taster 20 erstreckt (siehe Figuren 3B und 5). Dadurch wird der erste Taster 19 beim Betätigen des zweiten Tasters 20 mitbetätigt, sodass zuerst der Stilettschlitten 6 ausgelöst wird und sich nach vorne bewegt. Während dieser Bewegung ist der Kanülenschlitten jedoch noch durch den zweiten Kanülenschnapphaken 25 gehalten, der an der Kanülenschlittenhaltefläche 26 anliegt und durch den zweiten Taster 20 nicht betätigt werden kann. Gleichzeitig wird der erste Kanülenschnapphaken 17 jedoch durch die Betätigung des zweiten Tasters 20 von der ersten Kanülenschlittenhaltefläche 18 gelöst, sodass Kanülenschlitten 5 nur noch von dem zweiten Kanülenschnapphaken 25 gehalten wird.

Wenn sich nun der Stilettschlitten 6 weiter nach vorne bewegt, dann gelangt das Auslöseelement 27 über den zweiten Kanülenschnapphaken 25 und drückt diesen nach unten, sodass er von der zweiten Kanülenschlittenhaltefläche 26 gelöst wird, wenn der Stilettschlitten 6 an seiner entspannten Ausgangsposition angekommen ist. Somit wird der Kanülenschlitten 5 automatisch ausgelöst, wenn sich der Stilettschlitten entsprechend weit nach vorne bewegt hat.

Wie bei der ersten Art taucht auch hier die Nase 28 in die Vertiefung 29 ein. Der Grund dafür ist folgender.

Die Biopsiepistole 1 kann als Einweggerät ausgelegt werden, wobei die wesentlichen Bauteil Spritzgussbauteile sind, die so leicht und materialsparend wie möglich ausgebildet sind. Gleichzeitig können im Gebrauch der Biopsiepistole bspw. bei einer Prostatabiopsie große Belastungen aufgebracht werden, die dazu führen können, dass die Bestandteile sich verbiegen. Außerdem muss der zweite Kanülenschnapphaken 25 so ausgelegt sein, dass er einerseits sicher von der Schrägfläche am Bauteil, das die zweite Kanülenschlittenhaltefläche 26 aufweist, verformt wird, um danach an der zweiten Kanülenschlittenhaltefläche 26 einzurasten, andererseits muss er sicher von dem Auslöseelement 27 betätigt, also nach unten gebogen werden können. Je nach den Steifigkeits- und Belastungsverhältnissen kann jedoch der Fall eintreten, dass nicht der Kanülenschnapphaken 25 von dem Auslöseelement 27 nach unten gedrückt wird, sondern dass Kanülenschnapphaken 25 das Auslöseelement 27 nach oben drückt und somit verhindert wird, dass der Kanülenschnapphaken 25 von der Kanülenschlittenhaltefläche 26 gelöst wird. Dies würde dazu führen, dass der Kanülenschlitten nicht ausgelöst wird.

Durch das Eintauchen der Nase 28 am Auslöseelement 27 in die Vertiefung 29, die Teil der oberen Gehäusehalbschale ist, wird sichergestellt, dass der Stilettschlitten 6, insbesondere der Abschnitt, der das Auslöseelement 27 aufweist, mit Bezug auf die Vertikalrichtung in seiner Lage gehalten wird. Dadurch kann verhindert werden, dass dieser Abschnitt nach oben ausweicht, sodass der Kanülenschnapphaken 25 stets sicher gelöst wird.

Wie bereits erläutert wurde, besteht das Gehäuse 4 aus zwei Gehäusehalbschalen 4a und 4b mit L-förmigen Rahmenelementen 4a1 und 4b1, an deren vorderen Enden jeweils ein halbzylinderförmiger Abschnitt ausgebildet ist, die zusammen einen Stummelkegel 4c bilden, der als ein Kanülenführungsabschnitt dient. An dieser Stelle ist das Gehäuse besonders starken Belastungen ausgesetzt, da sich die Kanüle 2 und das Stilett 3 bspw. bei einer Prostatabiopsie stark verbiegen können und somit seitliche Kräfte auf den Stummelkegel 4c aufbringen, die auf die Innenseite des Stummelkegels 4c radial auswärts wirken. Ohne weiter Maßnahmen besteht daher die Gefahr, dass sich die Gehäusehalbschalen hier voneinander lösen, sodass eine einwandfreie Handhabung des Geräts nicht mehr gewährleistet ist.

Daher sind die beiden Rahmenelement 4a1 und 4b1 im Bereich des Stummelkegels 4c in beiden zueinander entgegengesetzten Längsrichtungen miteinander verrastet. Dazu hat das L- förmige Rahmenelement 4a1 an dem innenliegenden Ende seines kurzen Schenkels Vorsprünge 41 an beiden Seiten einer Aussparung, durch die die Kanüle 2 geführt ist (siehe Fig. 2a). An der vorderen Seite des kurzen Schenkels (siehe Fig. 2b) ist die eine Hälfte des Kegelstummels 4c ausgebildet, wobei sich zwei aufgegabelte Abschnitte des kurzen Schenkels mit nach hinten abgesenkten Niveau weiter Richtung Rahmenelement 4b1 erstrecken. In jedem aufgegabelten Abschnitt ist an der Vorderseite eine zur Innenseite zwischen den aufgegabelten Abschnitten offene Vertiefung 42 ausgebildet.

Fig. 5 zeigt die Gestaltung des kurzen Schenkels des L-förmigen Rahmenelements 4b1. Hier ist die zweite Hälfte des Kegelstummels 4c am Ende des kurzen Schenkels ausgebildet. Die nach hinten zeigende Fläche dieses Endes hat einen Vorsprung 43, der in die beiden Vertiefungen 42 des Rahmenelements 4a1 passt. Durch Zusammenwirken von Vorsprung 43 und Vertiefungen 42 sind die Enden der kurzen Schenkel der Rahmenelemente 4a1 und 4b1 in allen Richtungen senkrecht zur Längsachse aneinandergehalten.

Außerdem hat das Ende des kurzen Schenkels des Rahmens 4b1 einen Vorsprung 44, der sich an der hinteren Seite dieses Endes in Richtung des Rahmenelements 4a1 erstreckt und mit dem Vorsprung 41 des Rahmenelements 4a1 in Anlage gebracht werden kann. Somit sind die beiden Rahmenelemente 4a1 und 4b1 an deren Enden in der ersten Längsrichtung der Biopsiepistole 1 verrastet. Ferner liegt der Bereich des Endes des kurzen Schenkels des Rahmenelements 4b1 vor den aufgegabelten Abschnitten des Endes des kurzen Schenkels des Rahmenelements 4a1. Somit sind die beiden Rahmenelemente 4a1 und 4b1 an deren Enden in der zweiten Längsrichtung, die der ersten Längsrichtung entgegengesetzt ist, verrastet.

Somit kann der Kegelstummel 4c im montierten Zustand starken Belastungen durch die gebogene Kanüle ausgesetzt werden, ohne dass sich die Rastverbindung ungewollt löst.

Die Gehäusehalbschalen 4a, 4b haben zudem weitere Verrastungselemente, die die Gehäusehalbschalen 4a, 4b in Längsrichtung und Querrichtung zuverlässig miteinander verbindet.

## Patentansprüche

1. Biopsiepistole (1) mit einer Kanüle (2) und einem in der Kanüle (2) geführten Stilett (3), wobei die Biopsiepistole (1) ferner folgendes aufweist:
ein Gehäuse (4),
einen Kanülenschlitten (5) zum Bewegen der Kanüle (2),
einen Stilettschlitten (6) zum Bewegen des Stiletts (3),
wobei der Kanülenschlitten (5) und der Stilettschlitten (6) durch einen Spanngriff (7) von einer Ausgangsposition gegen jeweilige Federn in eine Spannposition gespannt werden können, wobei
der Stilettschlitten (6) einen Stilettschnapphaken (15) hat, der im gespannten Zustand des Stilettschlittens (6) mit einer Stilettschlittenhaltefläche (16) des Gehäuses (4) in Eingriff ist, wobei
der Kanülenschlitten (5) einen Kanülenschnapphaken (17) hat, der im gespannten Zustand des Kanülenschlittens (5) mit einer Kanülenschlittenhaltefläche (18) des Gehäuses (4) in Eingriff ist, wobei
das Gehäuse (4) einen ersten Taster (19) hat, durch dessen Betätigung der Eingriff des Stilettschnapphakens (15) mit der Stilettschlittenhaltefläche (16) durch Kontakt mit dem ersten Taster (19) gelöst wird, und einen zweiten Taster (20) hat, durch dessen Betätigung der Eingriff des Kanülenschnapphakens (17) mit der Kanülenschlittenhaltefläche (18) durch Kontakt mit dem zweiten Taster (20) gelöst wird, wobei
der erste Taster (19) eine Nase (21) hat, die unter den zweiten Taster (20) vorragt, sodass eine Betätigung des zweiten Tasters (20) zu einer Betätigung des ersten Tasters (19) führt, wobei
der erste und der zweite Taster (19, 20) über jeweilige Hauptverbindungselemente (22, 23) elastisch an dem Gehäuse (4) angeschlossen sind **dadurch gekennzeichnet, dass** der erste Taster (19) über ein zusätzliches Verbindungselement (24) an dem Gehäuse (4) elastisch angebunden ist, wobei das zusätzliche Verbindungselement (24) an einer Stelle des ersten Tasters (19) angeordnet ist, die der Stelle entgegengesetzt ist, an der der erste Taster (19) beim Betätigen mit dem Stilettschnapphaken (15) in Kontakt kommt.

2. Biopsiepistole (1) gemäß Anspruch 1, wobei
die Stelle des zusätzlichen Verbindungselements (24) des ersten Tasters (19) näher an dem Hauptverbindungselement (22) des ersten Tasters (19) als die Stelle liegt, an der der erste Taster (19) beim Betätigen mit dem Stilettschnapphaken (15) in Kontakt kommt.

## Claims

1. A biopsy gun (1) having a cannula (2) and a stylet (3) guided in the cannula (2), the biopsy gun (1) further comprising:
a housing (4),
a cannula carriage (5) for moving the cannula (2),
a stylet carriage (6) for moving the stylet (3),
wherein the cannula carriage (5) and the stylet carriage (6) can be tensioned by a tensioning grip (7) from an initial position into a tensioning position against respective springs, wherein
the stylet carriage (6) has a stylet snap-in hook (15) that is engaged with a stylet carriage holding surface (16) of the housing (4) when the stylet carriage (6) is in its tensioned state, wherein
the cannula carriage (5) has a cannula snap-in hook (17) that is engaged with a cannula carriage holding surface (18) of the housing (4) when the cannula carriage (5) is in its tensioned state, wherein
the housing (4) has a first key (19) by the operation of which the engagement of the stylet snap-in hook (15) with the stylet carriage holding surface (16) by contact with the first key (19) is released, and a second key (20) by the operation of which the engagement of the cannula snap-in hook (17) with the cannula carriage holding surface (18) is released by contact with the second key (20), wherein
the first key (19) has a lug (21) projecting under the second key (20), so that an operation of the second key (20) leads to an operation of the first key (19), wherein
the first and second keys (19, 20) are elastically connected to the housing (4) via respective main connecting elements (22, 23), **characterized in that** the first key (19) is elastically attached to the housing (4) via an additional connecting element (24), the additional connecting element (24) being arranged at a location of the first key (19) that is opposite to the location at which the first key (19), when operated, gets into contact with the stylet snap-in hook (15).

2. The biopsy gun (1) according to claim 1, wherein
the location of the additional connecting element (24) of the first key (19) is closer to the main connecting element (22) of the first key (19) than the location at which the first key (19), when operated, gets into contact with the stylet snap-in hook (15).

## Revendications

1. Pistolet à biopsie (1) comprenant une canule (2) et un stylet (3) guidé dans la canule (2), le pistolet à biopsie (1) présentant en outre :
un boîtier (4),
un chariot de canule (5) pour déplacer la canule (2),
un chariot de stylet (6) pour déplacer le stylet (3),
dans lequel le chariot de canule (5) et le chariot de stylet (6) peuvent être serrés par une poignée de serrage (7) d'une position initiale contre des ressorts respectifs à une position de serrage, dans lequel
le chariot de stylet (6) a un crochet d'enclenchement de stylet (15) qui, à l'état tendu du chariot de stylet (6), est en prise avec une surface de retenue de chariot de stylet (16) du boîtier (4), dans lequel
le chariot de canule (5) a un crochet d'enclenchement de canule (17) qui, à l'état tendu du chariot de canule (5), est en prise avec une surface de retenue de chariot de canule (18) du boîtier (4), dans lequel
le boîtier (4) a un premier palpeur (19), par l'actionnement duquel la prise du crochet d'enclenchement de stylet (15) avec la surface de retenue de chariot de stylet (16) est supprimée par contact avec le premier palpeur (19), et un deuxième palpeur (20) par l'actionnement duquel la prise du crochet d'enclenchement de canule (17) avec la surface de retenue de chariot de canule (18) est supprimée par contact avec le deuxième palpeur (20), dans lequel
le premier palpeur (19) a un nez (21) qui fait saillie sous le deuxième palpeur (20) de telle sorte qu'un l'actionnement du deuxième palpeur (20) conduit à un actionnement du premier palpeur (19), dans lequel
le premier et le deuxième palpeur (19, 20) sont raccordés de manière élastique au boîtier (4) par l'intermédiaire d'éléments de liaison principaux respectifs (22, 23), **caractérisé en ce que** le premier palpeur (19) est relié de manière élastique au boîtier (4) par l'intermédiaire d'un élément de liaison supplémentaire (24), dans lequel l'élément de liaison supplémentaire (24) est agencé à un emplacement du premier palpeur (19) qui est opposé à l'emplacement où le premier palpeur (19) vient en contact avec le crochet d'enclenchement de stylet (15) lors de l'actionnement.

2. Pistolet à biopsie (1) selon la revendication 1, dans lequel l'emplacement de l'élément de liaison supplémentaire (24) du premier palpeur (19) est situé plus près de l'élément de liaison principal (22) du premier palpeur (19) que l'emplacement où le premier palpeur (19) vient en contact avec le crochet d'enclenchement de stylet (15) lors de l'actionnement.
